(19) Europäisches Patentamt
European Patent Office
Office européen des brevets

(11) **EP 3 146 314 B1**

(12) **FASCICULE DE BREVET EUROPEEN**

(45) Date de publication et mention
de la délivrance du brevet:
**25.04.2018 Bulletin 2018/17**

(21) Numéro de dépôt: **15726047.2**

(22) Date de dépôt: **20.05.2015**

(51) Int Cl.:
*G01J 3/443* [(2006.01)]   *G01N 21/71* [(2006.01)]
*G01N 21/85* [(2006.01)]   *G01N 33/20* [(2006.01)]

(86) Numéro de dépôt international:
**PCT/EP2015/061136**

(87) Numéro de publication internationale:
**WO 2015/177223 (26.11.2015 Gazette 2015/47)**

(54) **DISPOSITIF D'ANALYSE D'UN MÉTAL EN FUSION OXYDABLE PAR TECHNIQUE LIBS**

VORRICHTUNG ZUR ANALYSE EINES OXIDIERBAREN GESCHMOLZENEN METALLS MIT EINER LIBS-TECHNIK

DEVICE FOR ANALYSING AN OXIDISABLE MOLTEN METAL USING A LIBS TECHNIQUE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.05.2014 FR 1454694**

(43) Date de publication de la demande:
**29.03.2017 Bulletin 2017/13**

(73) Titulaires:
• **Commissariat à l'Énergie Atomique et aux Énergies Alternatives**
**75015 Paris (FR)**
• **Université Pierre et Marie Curie (Paris 6)**
**75252 Paris Cedex 05 (FR)**

(72) Inventeurs:
• **BENMANSOUR, Malek**
**73290 La Motte-Servolex (FR)**
• **BENRABBAH, Rafik**
**75020 Paris (FR)**
• **GARANDET, Jean-Paul**
**92210 Saint Cloud (FR)**
• **MORVAN, Daniel**
**75005 Paris (FR)**

(74) Mandataire: **Brevalex**
**95, rue d'Amsterdam**
**75378 Paris Cedex 8 (FR)**

(56) Documents cités:
WO-A1-2007/012440   WO-A2-02/063284
DE-A1- 4 443 407   DE-B3-102006 047 765
US-A1- 2009 262 345   US-B1- 6 590 200

## Description

### DOMAINE TECHNIQUE

La présente invention se rapporte au domaine général de l'analyse spectrale d'un matériau, notamment un matériau liquide, et plus précisément au domaine de l'analyse spectrale d'un échantillon d'un tel matériau mettant en oeuvre la spectrométrie plasma induite par laser (encore appelée LIBS pour « Laser Induced Breakdown Spectroscopy » en anglais).

L'invention est particulièrement concernée par le domaine de l'analyse spectrale en ligne par technique LIBS des métaux en fusion, et spécifiquement des métaux en fusion fortement oxydables, comme par exemple le silicium. Elle peut ainsi s'appliquer par exemple à l'analyse de la composition chimique du silicium liquide en fusion durant sa purification par des procédés métallurgiques, pour son utilisation ultérieure, par exemple dans des cellules photovoltaïques.

L'invention propose ainsi un dispositif d'analyse d'au moins un métal en fusion oxydable par technique LIBS, un ensemble comportant une enceinte d'au moins un métal en fusion oxydable et un tel dispositif d'analyse, ainsi qu'un procédé d'analyse associé.

### ÉTAT DE LA TECHNIQUE ANTÉRIEURE

Dans le cadre de l'élaboration des cellules photovoltaïques, le silicium est le matériau le plus couramment utilisé. Il intervient ainsi dans la fabrication des cellules photovoltaïques dites « cristallines », c'est-à-dire qui sont à base de cristaux de silicium ou de polycristaux de silicium.

Pour obtenir le matériau silicium à la pureté requise pour son application, il est possible d'utiliser des procédés métallurgiques (tels que par exemple la solidification dirigée, l'évaporation réactive, entre autres) dans lesquels le silicium métallurgique passe par une phase liquide en fusion, et est purifié par exploitation de propriétés physiques des impuretés du silicium (coefficients de partage entre phase liquide et phase solide ou liquide, propriétés de volatilité, par exemple) ou exploitation de propriétés de réactivité des impuretés du silicium (traitement par plasma, par exemple). Toutefois, afin d'obtenir le silicium à la pureté requise, la maîtrise de la cinétique de purification passe par une connaissance précise de l'évolution des concentrations en impuretés dans le matériau au cours du temps de traitement.

De façon habituelle, l'analyse des composants du matériau silicium au cours de son traitement de purification est réalisée par prélèvement, dans le métal en fusion, d'un échantillon à l'aide d'un contenant généralement en graphite dans lequel le silicium est solidifié, démoulé, puis envoyé pour son analyse. Néanmoins, cette procédure classique d'analyse du matériau n'est pas entièrement satisfaisante, et en particulier n'est pas adaptée pour le contrôle en continu de la pureté du métal en fusion, car elle exige des étapes de préparation préalables d'échantillons qui nécessitent du temps et présentent des coûts élevés.

Aussi, au vu de ces inconvénients, il est apparu souhaitable de pouvoir mettre au point un outil d'analyse en ligne des composants du matériau silicium, de façon à réduire ainsi le temps d'analyse, les coûts et à assurer un contrôle continu de la pureté du matériau au cours du temps.

Pour ce faire, dans la perspective de l'analyse chimique et du diagnostic d'un matériau donné, le laser constitue un outil privilégié car il permet d'effectuer des opérations de détection et d'identification dans des conditions d'environnement très variées. De plus, les mesures réalisées par une technique laser présentent de nombreux atouts, et peuvent permettre une analyse *in situ*, sans prélèvement ni contact, ainsi qu'une rapidité d'acquisition des informations et l'utilisation pour des analyses locales ou à distance.

Parmi les techniques existantes utilisant un laser, la spectrométrie plasma induite par laser (dénommée « technique LIBS » dans toute la description) constitue une méthode analytique physique bien connue, utilisée pour l'analyse des composants d'un matériau afin de le caractériser. La technique LIBS est typiquement utilisée pour permettre une analyse rapide, directe (sans préparation d'échantillons) et en ligne de matériaux sous forme solide, liquide ou gazeuse. Elle met ainsi en oeuvre l'ablation-laser d'un matériau pour créer un plasma, puis la technologie spectroscopique pour l'observation et l'analyse du spectre lumineux du plasma afin de déterminer les composants du matériau.

Plus précisément, la technique LIBS implique la focalisation d'une impulsion laser vers la surface d'un échantillon du matériau à analyser, qui provoque la formation d'un micro-plasma. Ce micro-plasma se forme quasiment de façon immédiate, c'est-à-dire alors que l'impulsion laser n'est pas terminée. A la fin de l'impulsion laser, les espèces atomiques et ioniques du micro-plasma se désexcitent et réémettent alors un rayonnement qu'un analyseur, i.e. un spectromètre, capte et traduit afin d'obtenir un spectre décrivant les espèces chimiques qui composaient l'échantillon.

Ainsi, la technique LIBS peut permettre l'identification, grâce à la longueur d'onde d'émission, et la quantification, grâce à l'intensité d'émission, des composants présents dans l'échantillon du matériau à analyser. De plus, la technique LIBS permettant de réaliser une analyse à distance, elle s'avère tout particulièrement adaptée pour l'analyse des matériaux à l'état fondu à haute température, et notamment pour l'analyse du silicium en fusion, et l'analyse des matériaux ne pouvant pas être manipulés car représentant un danger potentiel. Aussi, dans le cas des métaux en fusion, tels que le silicium présenté précédemment, la technique LIBS est susceptible de fournir en temps réel l'évolution de la composition chimique du matériau, et notamment la teneur en impuretés du silicium métallurgique en fusion au cours de ses différentes étapes de purification.

Diverses solutions ont ainsi déjà été envisagées dans l'art antérieur pour mettre au point l'analyse en ligne de métaux en fusion par le biais de la technique LIBS.

Ainsi, dans l'article intitulé « Impurity detection in solid and molten silicon by laser induced breakdown spectroscopy », Sarah Darwiche et al, 2012, Spectrochimica Acta Part B, Volume 74-75, pages 115-118, les auteurs ont énoncé le principe de base consistant à focaliser une impulsion laser sur la surface d'un bain en fusion de métal, en particulier du silicium, et à collecter le signal émis par le plasma généré à l'aide d'un télescope. Le dispositif optique de mesure et le laser sont alors placés à distance suffisante du bain de métal en fusion afin d'éviter un endommagement éventuel par les flux de chaleur. Dans le cadre de cet article, l'analyse du silicium en fusion a été réalisée à distance de manière non intrusive et sur de faibles quantités de matière, en milieu inerté avec de l'argon. Néanmoins, à plus grande échelle, cette méthode de mesure peut présenter un inconvénient majeur lié au fait que, dans le cas de métaux en fusion, la composition chimique de la surface analysée n'est généralement pas représentative de la composition du volume global du métal. En effet, de par la forte réactivité des métaux en fusion, et par exemple du silicium, le matériau présent à l'interface avec l'atmosphère subit généralement des phénomènes d'oxydation ou de nitruration qui conduisent à la génération de laitiers de surface. Du fait de la ségrégation des diverses impuretés entre le métal fondu et la phase oxydée, le laitier n'a généralement pas la même composition que le métal, ce qui remet donc en cause la fiabilité des mesures réalisées avec cette méthode.

Des systèmes d'analyse alternatifs ont également été développés afin de créer une surface d'analyse sur l'échantillon qui soit représentative du volume de métal en fusion, comme par exemple décrit dans la demande internationale WO 2007/012440 A1 ou WO 2007/128014 A1. Dans l'article intitulé « High temperature fiber optic laser-induced breakdown spectroscopy sensor for analysis of molten alloy constituents », Awadhesh K. Rai et al, octobre 2002, Review of Scientific Instruments, Volume 73, pages 3589-3599, n°10, il est présenté un dispositif d'analyse par technique LIBS qui permet d'approcher la tête de mesure à proximité d'un bain d'aluminium en fusion et de créer une surface libre pour l'analyse à l'aide d'une entretoise. Le dispositif de collecte est protégé par une coque en acier inoxydable. Néanmoins, ce dispositif n'est pas satisfaisant en ce qu'il présente notamment l'inconvénient de limiter l'analyse à des matériaux à bas point de fusion, typiquement de l'ordre de 660°C pour l'aluminium à la pression atmosphérique, alors que le silicium, par exemple, présente un point de fusion aux alentours de 1412°C à la pression atmosphérique.

La demande internationale WO 02/063284 A2 propose encore une autre solution qui prévoit de faire circuler le métal liquide à travers une cellule et d'en faire l'analyse de surface par technique LIBS au moyen d'un accès optique. Cette solution a été appliquée pour le zinc et l'aluminium, mais elle n'est pas efficace pour des métaux très oxydables comme le silicium ou le zirconium. En effet, dans le cas d'un métal fortement oxydable, une oxydation de surface peut se produire avec l'oxygène résiduel de la charge et rendre l'analyse de surface invalide.

La demande de brevet US 2003/0234928 A1 décrit quant à elle une solution complémentaire à la précédente, pour les métaux très oxydables. Selon son principe, l'extrémité d'un tube est plongée sous la surface du métal liquide à travers duquel un bullage par gaz inerte est réalisé pour permettre de renouveler la surface du métal liquide et de générer un volume d'analyse dans une atmosphère neutre. L'information optique du plasma est alors collectée par l'intermédiaire d'un jeu de miroirs et de fibres optiques. Toutefois, cette solution présente l'inconvénient majeur d'une instabilité de la surface à analyser qui est intimement liée à la dynamique de la bulle générée. Une synchronisation, difficile à réaliser, entre la fréquence d'analyse et la dynamique de formation de la bulle est alors nécessaire. En outre, la note technique intitulée « VAI-CON® Chem-A New Continuous Chemical Analysis System of Liquid Steel in Metallurgical Vessels », N. Ramaseder et al, février 2004, La metallurgia italiana, pages 60-63, présente une solution similaire dans le cadre de l'analyse d'un bain d'acier. Cependant, cette solution a été testée sur des fours pilotes et reste difficilement applicable à des fours de capacité industrielle car elle utilise un jeu de miroirs dont le réglage est très sensible.

## EXPOSÉ DE L'INVENTION

Il existe par conséquent un besoin pour proposer une solution alternative pour permettre l'analyse en ligne par technique LIBS de métaux en fusion oxydables, et particulièrement fortement oxydables. Il existe notamment un besoin pour concevoir une solution simple de mise en oeuvre, robuste et permettant de fournir des résultats fiables de mesure optique.

En outre, il existe un besoin pour la mise en place d'une solution permettant l'obtention d'une surface stationnaire pour réaliser l'analyse par technique LIBS du métal en fusion. Par « surface stationnaire », il faut comprendre que les propriétés de la surface servant à l'analyse sont reproductibles dans le temps (à savoir notamment son niveau, sa forme, sa composition, sa vitesse d'écoulement, entre autres). En particulier, la préparation et le renouvellement de la surface servant à l'analyse doivent permettre d'éviter la formation d'une tierce phase (laitier, oxyde, nitrure, par exemple) qui pourrait invalider les résultats de l'analyse du métal.

L'invention a donc pour but de remédier au moins partiellement aux besoins mentionnés ci-dessus et aux inconvénients relatifs aux réalisations de l'art antérieur.

Elle vise notamment à proposer une telle solution pour permettre le contrôle en continu des concentrations des impuretés contenues dans du métal liquide en fusion oxydable, notamment du silicium, lors d'un processus de purification du métal.

L'invention a ainsi pour objet, selon l'un de ses aspects, un dispositif d'analyse d'au moins un métal en fusion oxydable par technique LIBS comportant des moyens d'analyse par technique LIBS, caractérisé en ce qu'il comporte en outre des moyens de brassage mécanique rotatifs d'un bain liquide dudit au moins un métal en fusion oxydable, les moyens de brassage mécanique comportant :

- une partie centrale, destinée à être positionnée au-dessus du bain liquide dudit au moins un métal en fusion oxydable, comprenant une cavité interne formant une chambre d'analyse, la partie centrale comportant une première extrémité reliée aux moyens d'analyse par technique LIBS,
- une pluralité de pales de brassage mécanique, destinées à être partiellement immergées dans le bain liquide dudit au moins un métal en fusion oxydable, reliées à une deuxième extrémité de la partie centrale, opposée à la première extrémité de la partie centrale,

les moyens d'analyse par technique LIBS étant configurés pour permettre l'analyse de la surface dudit au moins un métal en fusion oxydable situé dans la portion au droit de la cavité interne de la partie centrale.

Grâce à l'invention, il peut ainsi être possible de fournir une solution alternative pour permettre l'analyse en ligne par technique LIBS de métaux en fusion, tout spécialement adaptée aux métaux oxydables, et notamment fortement oxydables, en comparaison avec les solutions de l'art antérieur présentées précédemment. En effet, le dispositif selon l'invention permet avantageusement un renouvellement de la surface du métal en fusion à analyser par mélange mécanique à l'aide des pales de brassage et, de plus, il prévoit également la présence d'une chambre d'analyse spécifique pour permettre la focalisation de l'impulsion laser utilisée dans la technique LIBS.

Le dispositif selon l'invention peut en outre comporter l'une ou plusieurs des caractéristiques suivantes prises isolément ou suivant toutes combinaisons techniques possibles.

Les moyens d'analyse par technique LIBS peuvent être compris dans une tête d'analyse LIBS située au niveau de la première extrémité de la partie centrale. De plus, les moyens de brassage mécanique peuvent former ensemble un brasseur mécanique couplé à la tête d'analyse LIBS.

De façon avantageuse, la partie centrale peut comporter un ou plusieurs orifices formés dans sa paroi délimitant la cavité interne et situés au-dessus des pales de brassage mécanique lorsque le dispositif est en place dans le bain liquide dudit au moins un métal en fusion oxydable, le ou les orifices étant destinés à permettre l'évacuation d'un excès de métal en fusion oxydable remontant dans la cavité interne de la partie centrale en dehors de cette cavité interne.

Avantageusement, la présence de tels orifices sur une portion de la partie centrale située au-dessus des pales de brassage mécanique peut permettre d'assurer le renouvellement de la surface à analyser dudit au moins un métal en fusion oxydable. En effet, le métal en fusion oxydable situé dans la portion au droit de la cavité interne de la partie centrale peut voir son niveau remonter le long de la paroi de la partie centrale délimitant la cavité interne, en particulier sous l'effet de la centrifugation et de la capillarité. De cette façon, la présence d'orifices permettant l'écoulement permanent en dehors de la cavité interne de l'excès dudit au moins un métal en fusion oxydable qui remonte le long de la paroi, peut permettre de garantir le renouvellement et la stabilisation du niveau de la surface à analyser.

La forme, les dimensions et/ou la vitesse de rotation des pales de brassage mécanique et/ou de la partie centrale peuvent être déterminées et ajustées en fonction de la stabilisation requise de la surface à analyser dudit au moins un métal en fusion oxydable.

Les pales de brassage mécanique peuvent présenter diverses formes, et préférentiellement être de forme hélicoïdale.

Les dimensions des pales de brassage mécanique peuvent être définies en fonction des dimensions de l'enceinte dans laquelle est située ledit au moins un métal en fusion oxydable à analyser, de sorte à pouvoir assurer un brassage efficace du bain liquide de métal en fusion.

Par ailleurs, la partie centrale se présente sous la forme d'un tube (ou arbre) creux, notamment sous la forme d'un tube cylindrique creux.

De façon avantageuse, la partie centrale peut jouer le rôle de chambre d'analyse et permettre la focalisation de l'impulsion laser envoyée par les moyens d'analyse par technique LIBS vers la surface à analyser du bain de métal liquide, et permettre aussi le confinement du micro-plasma qui se forme.

De façon préférentielle, la partie centrale peut être réalisée en graphite. De plus, la partie centrale peut être revêtue extérieurement d'une couche formant barrière de passivation, notamment vis-à-vis du silicium, par exemple une couche de carbure de silicium (SiC).

Les moyens d'analyse par technique LIBS peuvent comporter un laser apte à générer une impulsion laser vers la surface à analyser dudit au moins un métal en fusion oxydable, un jeu de miroirs permettant la focalisation de l'impulsion laser vers la surface à analyser, un télescope relié à une fibre optique permettant la collecte des émissions du micro-plasma formé par l'impulsion laser, et un spectromètre d'émission permettant l'analyse des émissions collectées.

La durée du pulse du laser peut être de l'ordre de la femtoseconde à la nanoseconde. De plus, le laser peut fonctionner à différentes longueurs d'ondes, par exemple comprises entre 266 nm et 1064 nm, et préférentiellement dans le domaine de l'infrarouge. Son énergie peut être supérieure à 100 mJ.

Le jeu de miroirs peut permettre la focalisation de l'impulsion laser vers la surface à analyser à une distance d'environ 2 m.

La focalisation de l'impulsion laser vers la surface à analyser peut permettre la création du micro-plasma dont les émissions sont collectées par le télescope. Le spectromètre d'émission, qui permet alors d'analyser les émissions collectées, peut par exemple être un monochromateur du type « Czerny-Turner », pourvu de réseaux de diffraction adaptés.

En outre, le dispositif d'analyse selon l'invention peut comporter un système de soufflage de gaz inerte, notamment de l'hélium ou de l'argon, au travers de la partie centrale.

De façon avantageuse, le soufflage de gaz inerte à l'intérieur de la partie centrale peut permettre d'éviter d'éventuelles contaminations de la surface à analyser du bain dudit au moins un métal en fusion oxydable, par exemple pour éviter une oxydation en cas de micro-fuites. En outre, le soufflage de gaz inerte peut également présenter l'avantage d'augmenter les limites de détection de l'analyse par la technique LIBS.

En outre, l'invention a encore pour objet, selon un autre de ses aspects, un ensemble, caractérisé en ce qu'il comporte :

- une enceinte comportant un bain d'au moins un métal en fusion oxydable,
- un dispositif d'analyse tel que défini précédemment, pour l'analyse dudit au moins un métal en fusion oxydable de l'enceinte.

L'enceinte peut également être dénommée creuset. Elle peut par exemple être réalisée en graphite.

Avantageusement, ledit au moins un métal en fusion peut être un métal fortement oxydable, étant notamment choisi parmi le silicium, le zirconium, le manganèse, l'aluminium ou le titane, entre autres.

Par ailleurs, l'ensemble peut également comporter des moyens de chauffage de l'enceinte comportant ledit au moins un métal en fusion oxydable.

L'enceinte et la partie centrale du dispositif d'analyse peuvent être préférentiellement réalisées dans un même matériau, notamment en graphite.

Le régime d'écoulement dudit au moins un métal en fusion oxydable situé dans la portion au droit de la cavité interne de la partie centrale peut être laminaire, cet écoulement étant caractérisé par un nombre de Reynolds Re compris entre 100 et 5000, et notamment entre 1000 et 2000, ce nombre de Reynolds Re étant donné par la formule suivante :

$$Re = [(\omega \times R) \times R']/v,$$

où :

- $(\omega \times R)$ représente la vitesse caractéristique de l'écoulement, à savoir le produit de la vitesse angulaire de rotation $\omega$ d'une pale de brassage mécanique et de la distance R entre l'extrémité de la pale de brassage mécanique et l'axe de la partie centrale,
- R' représente une dimension caractéristique de l'écoulement, à savoir notamment le rayon de la partie centrale dans le cas où elle est cylindrique ou le côté de la partie centrale dans le cas où elle est carrée par exemple, et
- v représente la viscosité cinématique du liquide.

Ainsi, le choix de la valeur du nombre de Reynolds Re effectué pour caractériser l'écoulement dudit au moins un métal en fusion oxydable situé dans la portion au droit de la cavité interne de la partie centrale peut donc avoir un impact direct sur les paramètres caractérisant l'intensité du brassage mécanique du bain de métal liquide.

Par ailleurs, l'invention a aussi pour objet, selon un autre de ses aspects, un procédé d'analyse d'au moins un métal en fusion oxydable par technique LIBS, caractérisé en ce qu'il est mis en oeuvre au moyen d'un dispositif d'analyse tel que défini précédemment, et en ce qu'il comporte les étapes simultanées consistant à :

- effectuer un brassage mécanique d'un bain liquide dudit au moins un métal en fusion oxydable par le biais des moyens de brassage mécanique rotatifs du dispositif,
- analyser par technique LIBS la surface dudit au moins un métal en fusion oxydable situé dans la portion au droit de la cavité interne de la partie centrale par le biais des moyens d'analyse par technique LIBS.

Le procédé peut en outre comporter au moins une étape d'analyse en ligne par technique LIBS d'une ou plusieurs impuretés contenues dans ledit au moins un métal en fusion oxydable, notamment du silicium, lors d'un processus de purification dudit au moins un métal en fusion oxydable.

Le dispositif, l'ensemble et le procédé selon l'invention peuvent comporter l'une quelconque des caractéristiques énoncées dans la description, prises isolément ou selon toutes combinaisons techniquement possibles avec d'autres caractéristiques.

**BRÈVE DESCRIPTION DES DESSINS**

L'invention pourra être mieux comprise à la lecture de la description détaillée qui va suivre, d'un exemple de mise en oeuvre non limitatif de celle-ci, ainsi qu'à l'examen des figures, schématiques et partielles, du dessin annexé, sur lequel :

- la figure 1 représente, en coupe, un exemple de dispositif d'analyse d'un métal en fusion oxydable par technique LIBS conforme à l'invention, et
- la figure 2 représente une vue agrandie de la zone A de la figure 1.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

## EXPOSÉ DÉTAILLÉ D'UN MODE DE RÉALISATION PARTICULIER

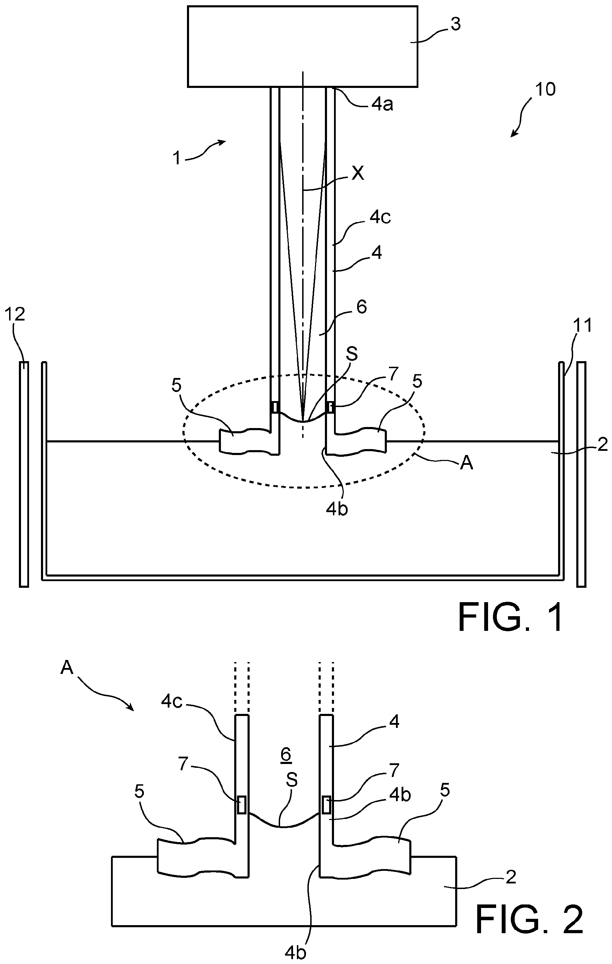

Dans l'exemple décrit ci-après en référence aux figures 1 et 2, on considère que le métal 2 en fusion oxydable correspond à du silicium, et notamment du silicium métallurgique. Le dispositif 1 d'analyse selon l'invention peut alors être utilisé pour le contrôle en continu des concentrations des impuretés contenues dans ce silicium liquide en fusion, lors d'un processus de purification du métal visant par exemple à permettre une utilisation ultérieure pour l'élaboration de cellules photovoltaïques. Bien entendu, ce choix n'est nullement limitatif. En particulier, l'invention pourrait avantageusement s'appliquer à d'autres types de métaux en fusion oxydables, et notamment fortement oxydables, comme par exemple le zirconium, dont l'analyse de surface nécessite un renouvellement permanent de la matière afin de garantir une fiabilité acceptable des résultats de mesure.

On se réfère ainsi à la figure 1 qui représente, en coupe, un exemple de dispositif 1 d'analyse d'un métal 2 en fusion oxydable, à savoir le silicium, par technique LIBS conforme à l'invention. Par ailleurs, la figure 2 représente une vue agrandie de la zone A de la figure 1.

Conformément à l'invention, le dispositif 1 d'analyse comporte des moyens d'analyse par technique LIBS 3 et des moyens de brassage mécanique 4, 5 rotatifs d'un bain liquide de silicium 2 en fusion. Ce bain liquide de silicium 2 comporte par exemple une charge de silicium de grade métallurgique amélioré (encore dénommée UMG-Si pour « Upgraded metallurgical-Grade Silicon » en anglais), qui comporte une composition chimique d'environ 300 ppm en masse de métaux tous confondus, environ 15 ppm en masse de bore et environ 20 ppm en masse de phosphore.

Plus spécifiquement, le dispositif 1 d'analyse est utilisé dans un ensemble 10 conforme à l'invention qui comporte, en plus du dispositif 1 d'analyse, une enceinte 11, couramment dénommée creuset, et par exemple réalisée en silice et revêtue de nitrure de silicium, comprenant le bain de silicium 2 liquide en fusion. En particulier, l'utilisation du dispositif 1 d'analyse selon l'invention peut être faite dans cet exemple sur une opération de ségrégation dans un four de solidification dirigée d'une capacité d'environ 60 kg. En outre, afin de permettre le chauffage du silicium 2 dans l'enceinte 11 et le maintien d'une température supérieure à sa température de fusion d'environ 1412 °C à la pression atmosphérique, des moyens de chauffage résistifs 12 de l'enceinte 11 sont également prévus. De cette façon, la charge de silicium 2 peut par exemple être fondue sous un flux d'argon par chauffage résistif.

Par ailleurs, les moyens de brassage mécanique 4, 5 forment ensemble un brasseur mécanique 4, 5, couplé aux moyens d'analyse par technique LIBS 3 situés dans une tête d'analyse LIBS 3.

Ce brasseur mécanique 4, 5 comporte une partie centrale 4 située au-dessus du bain liquide de silicium 2 en fusion, qui comprend une cavité interne 6 formant une chambre d'analyse. De plus, la partie centrale 4 comporte une première extrémité 4a qui est reliée à la tête d'analyse LIBS 3.

La partie centrale 4 se présente sous la forme d'un tube de brassage creux cylindrique, pourvu d'une paroi annulaire 4c délimitant la cavité interne 6, et présente par exemple un diamètre intérieur d'environ 25 mm et un diamètre extérieur d'environ 65 mm.

La partie centrale 4 joue le rôle de chambre d'analyse et permet la focalisation de l'impulsion laser envoyée par la tête d'analyse LIBS 3 vers la surface S à analyser du bain de silicium 2. Cette partie centrale 4 est notamment réalisée en graphite, et revêtue extérieurement d'une couche formant barrière de passivation vis-à-vis du silicium, par exemple une couche de carbure de silicium.

De plus, le brasseur mécanique 4, 5 comporte également des pales de brassage mécanique 5 immergées partiellement dans le bain de silicium 2, et reliées à une deuxième extrémité 4b de la partie centrale 4. Les pales de brassage mécanique 5 sont par exemple de forme hélicoïdale et leur distance par rapport à l'axe central X de la partie centrale 4 est d'environ 50 mm.

Lorsque le silicium 2 est en fusion complète, la partie centrale 4 est introduite progressivement dans le bain de silicium 2 avec immersion partielle des pales de brassage 5, puis grâce à un moteur, elle est mise en rotation pour assurer le brassage du bain de silicium 2. L'analyse par technique LIBS est alors réalisée sur la surface S du silicium 2 situé dans la portion au droit de la partie centrale 4.

La rotation de la partie centrale 4 peut par exemple se faire à l'aide de l'empilement d'au moins deux pignons fixés autour de l'axe central X de la partie centrale 4. La vitesse de rotation du brasseur mécanique 4, 5 peut par exemple être fixée aux alentours de 10 tours/min, ce qui correspond à un nombre de Reynolds $Re$ d'environ 1870. De cette façon, il peut être possible d'obtenir un écoulement laminaire du silicium 2 au droit de la partie centrale 4, et une circulation facilitée du silicium 2 au travers des orifices 7 prévus sur la partie centrale 4, comme il sera décrit par la suite.

De façon plus générale, il est avantageux d'obtenir un régime d'écoulement du silicium 2 situé au droit de la cavité interne 6 de la partie centrale 4 qui soit laminaire. Pour ce faire, le nombre de Reynolds *Re* est préférentiellement compris entre 100 et 5000, et notamment entre 1000 et 2000, ce nombre de Reynolds *Re* étant donné par la formule suivante : $Re = [(\omega \times R) \times R']/v$, dans laquelle : $(\omega \times R)$ représente la vitesse caractéristique de l'écoulement, à savoir le produit de la vitesse angulaire de rotation $\omega$ d'une pale de brassage mécanique 5 et de la distance R entre l'extrémité de la pale de brassage mécanique 5 et l'axe X de la partie centrale 4, R' représente une dimension caractéristique de l'écoulement, à savoir le rayon de la partie centrale 4, et v représente la viscosité cinématique du liquide.

Par ailleurs, comme il est davantage visible sur la figure 2, la partie centrale 4 comporte des orifices 7 formés dans sa paroi annulaire 4c, qui sont situés au-dessus des pales de brassage mécanique 5 lorsque le dispositif 1 est en place dans le bain liquide de silicium 2. Ces orifices 7 permettent l'évacuation d'un excès de silicium 2 qui remonterait dans la cavité interne 6 de la partie centrale 2, en dehors de cette cavité interne 6. En effet, le silicium 2 situé au droit de la cavité interne 6 peut voir son niveau remonter le long de la paroi 4c de la partie centrale 4 sous l'effet de la centrifugation et de la capillarité. De cette façon, la présence d'orifices 7 permet d'assurer le renouvellement de la surface S à analyser et le maintien du niveau de silicium 2 à un niveau constant dans la cavité interne 6.

Par ailleurs, la tête d'analyse LIBS 3 peut comporter un laser apte à générer une impulsion laser vers la surface S à analyser du silicium 2, ce laser étant par exemple du type Nd-YAG nano pulsé (de l'acronyme anglais « neodymium-doped yttrium aluminium garnet »), avec une durée d'impulsion de l'ordre de 5 ns, fonctionnant à une longueur d'onde d'environ 1064 nm. Ce laser peut permettre de délivrer des impulsions laser d'une énergie maximale d'environ 200 mJ. Le signal est alors récupéré par le biais d'un télescope, puis focalisé à l'entrée d'un monochromateur à l'aide d'un faisceau de fibres optiques. Le monochromateur utilisé est par exemple composé d'un réseau de diffraction de 2400 traits.mm$^{-1}$ et d'un détecteur i-CCD permettant un pilotage temporel de l'analyse. En effet, pour l'analyse, il s'agit de déterminer un délai à partir duquel l'analyse débute et une durée d'accumulation définie comme la porte d'analyse.

A titre d'exemple, une analyse a été réalisée avec le dispositif 1 d'analyse conforme à l'invention, en utilisant une énergie d'impulsion laser de l'ordre de 30 mJ, un délai d'impulsion de l'ordre de 1 $\mu$s, et une durée d'accumulation du signal de l'ordre de 5 $\mu$s. De plus, pour améliorer l'intensité du signal, et donc la limite de détection, un flux d'argon a été soufflé sur la surface S du bain de silicium 2 en fusion, par le biais d'un système de soufflage de gaz inerte, au travers de l'axe creux de la partie centrale 4 avec un débit d'environ 1 L.min$^{-1}$.

Une mesure a alors été réalisée dans le silicium 2 tout le long d'une solidification dirigée. Elle a permis de déterminer une concentration initiale Co des différentes impuretés, puis de connaître leurs évolutions au cours du temps dans le bain de silicium 2. Cette mesure a permis la détection des principales impuretés métalliques du silicium métallurgique, à savoir l'aluminium, le calcium et le fer, ainsi que les dopants que sont le bore et le phosphore. Dans le cas des impuretés métalliques, il a été possible d'obtenir une limite de détection d'environ 0,1 ppm en masse, alors que celle-ci a été de l'ordre d'1 ppm en masse pour le bore et de 10 ppm en masse pour le phosphore.

Par conséquent, par son caractère non intrusif et ne nécessitant pas de préparation d'échantillons, l'analyse du métal 2 en fusion par la technique LIBS permet avantageusement d'apporter une information instantanée sur la pureté du silicium 2 à l'état liquide au cours de sa purification. L'utilisation des moyens de brassage mécanique 4, 5 du dispositif 1 selon l'invention permet en outre d'assurer le renouvellement de la surface S à analyser du bain de métal 2 liquide pendant la mesure par technique LIBS. De plus, un réglage optimal de la vitesse de rotation et des dimensions des pales de brassage 5 et de la partie centrale 4 permet d'assurer un échantillonnage représentatif du métal 2 en fusion, mais aussi de garantir le caractère stationnaire du niveau de la surface S à analyser pour une meilleure focalisation sur l'axe central X de la partie centrale 4 de l'impulsion laser des moyens d'analyse par technique LIBS 3.

L'invention peut ainsi rendre possible le suivi en ligne de la composition du métal 2 en fusion au cours de sa purification, effectuée notamment par des procédés métallurgiques tels que la solidification dirigée ou encore l'évaporation réactive. En conséquence, il peut ainsi être possible de contrôler la qualité du matériau obtenu et d'ajuster les temps de cycle en cours de traitement, permettant également d'obtenir un gain en termes de productivité et une réduction des coûts de fabrication.

Bien entendu, l'invention n'est pas limitée à l'exemple de réalisation qui vient d'être décrit. Diverses modifications peuvent y être apportées par l'homme du métier.

L'expression « comportant un » doit être comprise comme étant synonyme de « comportant au moins un », sauf si le contraire est spécifié.

## Revendications

1. Dispositif (1) d'analyse d'au moins un métal (2) en fusion oxydable par technique LIBS comportant des moyens d'analyse par technique LIBS (3), **caractérisé en ce qu'**il comporte en outre des moyens de brassage mécanique (4, 5) rotatifs d'un bain liquide dudit au moins un métal (2) en fusion oxydable, les moyens de brassage mécanique (4, 5) comportant :

- une partie centrale (4), se présentant sous la forme d'un tube creux et destinée à être positionnée au-dessus du bain liquide dudit au moins un métal (2) en fusion oxydable, comprenant une cavité interne (6) formant une chambre d'analyse, la partie centrale (4) comportant une première extrémité (4a) reliée aux moyens d'analyse par technique LIBS (3),
- une pluralité de pales de brassage mécanique (5), destinées à être partiellement immergées dans le bain liquide dudit au moins un métal (2) en fusion oxydable, reliées à une deuxième extrémité (4b) de la partie centrale (4), opposée à la première extrémité (4a) de la partie centrale (4),

les moyens d'analyse par technique LIBS (3) étant configurés pour permettre l'analyse de la surface (S) dudit au moins un métal (2) en fusion oxydable situé dans la portion au droit de la cavité interne (6) de la partie centrale (4).

2. Dispositif selon la revendication 1, **caractérisé en ce que** la partie centrale (4) comporte un ou plusieurs orifices (7) formés dans sa paroi (4c) délimitant la cavité interne (6) et situés au-dessus des pales de brassage mécanique (5) lorsque le dispositif (1) est en place dans le bain liquide dudit au moins un métal (2) en fusion oxydable, le ou les orifices (7) étant destinés à permettre l'évacuation d'un excès de métal (2) en fusion oxydable remontant dans la cavité interne (6) de la partie centrale (2) en dehors de cette cavité interne (6).

3. Dispositif selon la revendication 1 ou 2, **caractérisé en ce que** la partie centrale (4) se présente sous la forme d'un tube cylindrique creux.

4. Dispositif selon l'une des revendications précédentes, **caractérisé en ce que** les moyens d'analyse par technique LIBS (3) comportent un laser apte à générer une impulsion laser vers la surface (S) à analyser dudit au moins un métal (2) en fusion oxydable, un jeu de miroirs permettant la focalisation de l'impulsion laser vers la surface (S) à analyser, un télescope relié à une fibre optique permettant la collecte des émissions du micro-plasma formé par l'impulsion laser, et un spectromètre d'émission permettant l'analyse des émissions collectées.

5. Dispositif selon l'une quelconque des revendications précédentes, **caractérisé en ce qu'**il comporte en outre un système de soufflage de gaz inerte, notamment de l'hélium ou de l'argon, au travers de la partie centrale (4).

6. Ensemble (10), **caractérisé en ce qu'**il comporte :

- une enceinte (11) comportant un bain d'au moins un métal (2) en fusion oxydable,
- un dispositif (1) d'analyse selon l'une quelconque des revendications précédentes, pour l'analyse dudit au moins un métal (2) en fusion oxydable de l'enceinte (11).

7. Ensemble selon la revendication 6, **caractérisé en ce que** ledit au moins un métal (2) en fusion est un métal fortement oxydable, étant notamment choisi parmi le silicium ou le zirconium.

8. Ensemble selon la revendication 6 ou 7, **caractérisé en ce que** l'enceinte (11) et la partie centrale (4) du dispositif (1) d'analyse sont réalisées dans un même matériau, notamment en graphite.

9. Ensemble selon l'une quelconque des revendications 6 à 8, **caractérisé en ce que** le régime d'écoulement dudit au moins un métal (2) en fusion oxydable situé dans la portion au droit de la cavité interne (6) de la partie centrale (4) est laminaire, cet écoulement étant **caractérisé par** un nombre de Reynolds $Re$ compris entre 100 et 5000, et notamment entre 1000 et 2000, ce nombre de Reynolds $Re$ étant donné par la formule suivante :

$$Re = [(\omega \times R) \times R']/\nu,$$

où :

- ($w \times R$) représente la vitesse caractéristique de l'écoulement, à savoir le produit de la vitesse angulaire de rotation $\omega$ d'une pale de brassage mécanique (5) et de la distance R entre l'extrémité de la pale de brassage mécanique (5) et l'axe (X) de la partie centrale (4),
- R' représente une dimension caractéristique de l'écoulement, à savoir notamment le rayon de la partie centrale (4), et
- $\nu$ représente la viscosité cinématique du liquide.

10. Procédé d'analyse d'au moins un métal (2) en fusion oxydable par technique LIBS, **caractérisé en ce qu'**il est mis en oeuvre au moyen d'un dispositif (1) d'analyse selon l'une quelconque des revendications 1 à 5, et **en ce qu'**il comporte les étapes simultanées consistant à :

- effectuer un brassage mécanique d'un bain liquide dudit au moins un métal (2) en fusion oxydable par le biais des moyens de brassage mécanique (4, 5) rotatifs du dispositif (1),

- analyser par technique LIBS la surface (S) dudit au moins un métal (2) en fusion oxydable situé au droit de la cavité interne (6) de la partie centrale (4) par le biais des moyens d'analyse par technique LIBS (3).

11. Procédé selon la revendication 10, **caractérisé en ce qu'**il comporte au moins une étape d'analyse en ligne par technique LIBS d'une ou plusieurs impuretés contenues dans ledit au moins un métal (2) en fusion oxydable, notamment du silicium, lors d'un processus de purification dudit au moins un métal (2) en fusion oxydable.

**Patentansprüche**

1. Vorrichtung (1) zur Analyse von zumindest einer oxidierbaren Metallschmelze (2) durch LIBS-Technik, enthaltend LIBS-Analyseeinrichtungen (3), **dadurch gekennzeichnet, dass** es ferner drehbare Einrichtungen (4, 5) zum mechanischen Aufrühren eines flüssigen Bades der zumindest einen oxidierbaren Metallschmelze (2) enthält, wobei die Einrichtungen (4, 5) zum mechanischen Aufrühren enthalten:

  - einen Mittelabschnitt (4), der in Form eines Hohlrohrs vorliegt und dazu bestimmt ist, oberhalb des flüssigen Bades der zumindest einen oxidierbaren Metallschmelze (2) positioniert zu werden, enthaltend einen Innenhohlraum (6), der eine Analysekammer bildet, wobei der Mittelabschnitt (4) ein erstes Ende (4a) aufweist, das mit den LIBS-Analyseeinrichtungen (3) verbunden ist,
  - eine Mehrzahl von Rührblättern (5) zum mechanischen Aufrühren, die dazu bestimmt sind, teilweise in das flüssige Bad der zumindest einen oxidierbaren Metallschmelze einzutauchen, mit einem zweiten Ende (4b) des Mittelabschnitts (4) verbunden sind, das dem ersten Ende (4a) des Mittelabschnitts (4) entgegengesetzt ist,

  wobei die LIBS-Analyseeinrichtungen (3) dazu ausgelegt sind, die Analyse der Oberfläche (S) der zumindest einem oxidierbaren Metallschmelze (2) zu gestatten, die sich in dem Abschnitt im Bereich des Innenhohlraums (6) des Mittelabschnitts (4) befindet.

2. Vorrichtung nach Anspruch 1, **dadurch gekennzeichnet, dass** der Mittelabschnitt (4) eine oder mehrere Öffnungen (7) aufweist, die in seiner den Innenhohlraum (6) eingrenzenden Wand (4c) ausgebildet sind und oberhalb der Rührblätter (5) zum mechanischen Aufrühren liegen, wenn die Vorrichtung (1) in dem flüssigen Bad der zumindest einen oxidierbaren Metallschmelze (2) in Stellung ist, wobei die Öffnung bzw. Öffnungen (7) dazu bestimmt sind, das Ableiten eines Überschusses an oxidierbarer Metallschmelze (2) zu gestatten, der im Innenhohlraum (6) des Mittelabschnitts (2) nach außerhalb dieses Innenhohlraums (6) ansteigt.

3. Vorrichtung nach Anspruch 1 oder 2, **dadurch gekennzeichnet, dass** der Mittelabschnitt (4) in Form von einem zylindrischen Hohlrohr vorliegt.

4. Vorrichtung nach einem der vorangehenden Ansprüche, **dadurch gekennzeichnet, dass** die LIBS-Analyseeinrichtungen (3) einen Laser enthalten, der dazu geeignet ist, einen Laserimpuls zur zu analysierenden Oberfläche (S) der zumindest einen oxidierbaren Metallschmelze (2) zu erzeugen, wobei ein Satz von Spiegeln das Fokussieren des Laserimpulses zur zu analysierenden Oberfläche (S) gestattet, sowie ein mit einer optischen Faser verbundenes Teleskop, das das Erfassen von Emissionen des von dem Laserimpuls gebildeten Mikroplasmas gestattet, und ein Emisssionsspektrometer, das die Analyse der erfassten Emissionen gestattet.

5. Vorrichtung nach einem der vorangehenden Ansprüche, **dadurch gekennzeichnet, dass** es ferner ein System zum Blasen von Inertgas, insbesondere Helium oder Argon, durch den Mittelabschnitt (4) hindurch enthält.

6. Baugruppe (10), **dadurch gekennzeichnet, dass** sie enthält:

  - einen Behälter (11) mit einem Bad aus zumindest einer oxidierbaren Metallschmelze (2),
  - eine Analysevorrichtung (1) nach einem der vorangehenden Ansprüche zur Analyse der zumindest einen oxidierbaren Metallschmelze (2) des Behälters (11).

7. Baugruppe nach Anspruch 6, **dadurch gekennzeichnet, dass** die zumindest eine Metallschmelze (2) ein stark oxidierbares Metall ist, wobei es insbesondere ausgewählt ist aus Silicium oder Zirkonium.

8. Baugruppe nach Anspruch 6 oder 7, **dadurch gekennzeichnet, dass** der Behälter (11) und der Mittelabschnitt (4) der Analysevorrichtung (1) aus einem gleichen Material, insbesondere aus Graphit, hergestellt sind.

9. Baugruppe nach einem der Ansprüche 6 bis 8, **dadurch gekennzeichnet, dass** die Strömungsgeschwindigkeit der zumindest einen oxidierbaren Metallschmelze (2), die in dem Teil im Bereich des Innenhohlraums (6) des Mittelabschnitts (4) liegt, laminar ist, wobei diese Strömung durch eine Reynolds-Zahl *Re* zwischen 100 und 5000 und insbesondere zwischen 1000 und 2000 gekennzeichnet ist, wobei diese Reynolds-Zahl *Re* durch die nachfolgende Formel gegeben ist:

$$Re = [(\omega \times R) \times R'] / v$$

worin:

- ($\omega$ x R) die charakteristische Strömungsgeschwindigkeit darstellt, nämlich das Produkt der Rotationswinkelgeschwindigkeit $\omega$ eines Rührblatts (5) zum mechanischen Aufrühren und dem Abstand R zwischen dem Ende des Rührblatts (5) und der Achse (X) des Mittelabschnitts (4),
- R' eine charakteristische Strömungsabmessung darstellt, nämlich insbesondere den Radius des Mittelabschnitts (4), und
- v die kinematische Viskosität der Flüssigkeit darstellt.

10. Verfahren zur Analyse zumindest einer oxidierbaren Metallschmelze (2) durch LIBS-Technik, **dadurch gekennzeichnet, dass** es mittels einer Analysevorrichtung (1) nach einem der Ansprüche 1 bis 5 durchgeführt wird und dass es die nachfolgenden, gleichzeitigen Schritte umfasst:

- mechanisches Aufrühren eines flüssigen Bades der zumindest einen oxidierbaren Metallschmelze (2) über drehbare Rühreinrichtungen (4, 5) der Vorrichtung (1),
- Analysieren der Oberfläche (S) der zumindest einen oxidierbaren Metallschmelze (2) durch LIBS-Technik, die sich im Bereich des Innenhohlraums (6) des Mittelabschnitts (4) befindet, über die LIBS-Analyseeinrichtungen (3).

11. Verfahren nach Anspruch 10, **dadurch gekennzeichnet, dass** es zumindest einen Schritt der Inline-Analyse mittels LIBS-Technik von einer oder mehreren Unreinheiten, die in der zumindest einen oxidierbaren Metallschmelze (2), insbesondere Silicium, enthalten sind, bei einem Reinigungsprozess zum Reinigen der zumindest einen oxidierbaren Metallschmelze (2) umfasst.

**Claims**

1. Device (1) for the analysis of at least one oxidisable molten metal (2) based on the LIBS technique comprising analysis means using the LIBS technique (3), **characterised in that** it also comprises mechanical rotating means (4, 5) for stirring the liquid bath of said at least one oxidisable molten metal (2), the mechanical stirring means (4, 5) comprising:

- a central part (4) in the form of a hollow tube, that will be positioned above the liquid bath of said at least one oxidisable molten metal (2), comprising an internal cavity (6) forming an analysis chamber, the central part (4) comprising a first end (4a) connected to the analysis means using the LIBS technique (3),
- a plurality of mechanical stirring paddles (5) that will be partially immersed in the liquid bath of said at least one oxidisable molten metal (2), connected to a second end (4b) of the central part (4), opposite the first end (4a) of the central part (4), the means of analysis using the LIBS technique (3) being configured to enable the analysis of the surface (S) of said at least one oxidisable molten metal (2) located in the portion adjacent to the internal cavity (6) of the central part (4).

2. Device according to claim 1, **characterised in that** the central part (4) comprises one or several orifices (7) formed in its wall (4c) delimiting the internal cavity (6) and located above the mechanical stirring paddles (5) when the device (1) is placed in the liquid bath of said at least one oxidisable molten metal (2), the orifice(s) (7) being designed to evacuate excess oxidisable molten metal (2) brought up into the internal cavity (6) of the central part (2) outside this internal cavity (6).

3. Device according to claim 1 or 2, **characterised in that** the central part (4) is in the form of a hollow cylindrical tube.

4. Device according to any one of the previous claims, **characterised in** the means of analysis using the LIBS technique (3) comprise a laser capable of generating a laser pulse towards the surface (S) to be analysed of said at least one oxidisable molten metal (2), a set of mirrors for focussing the laser pulse to the surface (S) to be analysed, a telescope connected to an optical fibre for the collection of emissions from the micro-plasma formed by the laser pulse, and an emission spectrometer for analysis of the collected emissions.

5. Device according to any one of the previous claims, **characterised in that** it also comprises a system for blowing inert gas, particularly helium or argon, through the central part (4).

6. Assembly (10), **characterised in that** it comprises

- a chamber (11) containing a bath of said at least one oxidisable molten metal (2),
- an analysis device (1) according to any one of the above claims, for the analysis of said at least one oxidisable molten metal (2) in the chamber (11).

7. Assembly according to claim 6, **characterised in that** said at least one molten metal (2) is a strongly oxidisable metal, chosen particularly from among silicon and zirconium.

8. Assembly according to claim 6 or 7, **characterised in that** the chamber (11) and the central part (4) of the analysis device (1) are made from the same material, particularly graphite.

9. Assembly according to any one of claims 6 to 8, **characterised in** the flow rate of said at least one oxidisable molten metal (2) located in the portion adjacent to the internal cavity (6) of the central part (4) is laminar, this flow being **characterised by** a Reynolds number *Re* equal to between 100 and 5000, and particularly between 1000 and 2000, this Reynolds number *Re* being given by the following formula:

$$Re = [(\omega \times R) \times R']/v,$$

in which:

- (w x R) represents the characteristic flow velocity, in other words the product of the angular rotation speed $\omega$ of a mechanical stirring paddle (5) and the distance R between the end of the mechanical stirring paddle (5) and the axis (X) of the central part (4),
- R' represents a dimension characteristic of the flow, namely in particular the radius of the central part (4), and
- v represents the kinematic viscosity of the liquid.

10. Method for analysing at least one oxidisable molten metal (2) using the LIBS technique, **characterised in that** it used an analysis device (1) according to any one of claims 1 to 5, and **in that** it comprises simultaneous steps to:

- mechanically stir a liquid bath of said oxidisable at least one molten metal (2) making use of rotating mechanical stirring means (4, 5) of the device (1),
- use the LIBS technique to analyse the surface (S) of said at least one oxidisable molten metal (2) located adjacent to the internal cavity (6) in the central part (4) using analyses means based on the LIBS technique (3).

11. Method according to claim 10, **characterised in that** it comprises at least one step of an in-line analysis using the LIBS technique of one or several impurities contained in said at least one oxidisable molten metal (2), particularly silicon, during a purification process of said at least one oxidisable molten metal (2).

FIG. 1

FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007012440 A1
- WO 2007128014 A1
- WO 02063284 A2
- US 20030234928 A1

**Littérature non-brevet citée dans la description**

- **SARAH DARWICHE et al.** *Spectrochimica Acta Part B,* 2012, vol. 74 (75), 115-118
- **AWADHESH K. RAI et al.** *Review of Scientific Instruments,* Octobre 2002, vol. 73 (10), 3589-3599
- **N. RAMASEDER et al.** VAI-CON® Chem-A New Continuous Chemical Analysis System of Liquid Steel in Metallurgical Vessels. *La metallurgia italiana,* Février 2004, 60-63